# EUROPEAN PATENT APPLICATION

(11) **EP 3 412 655 A1**
(43) Date of publication of application: **12.12.2018**
(21) Application number: 17174580.5
(22) Date of filing: 06.06.2017
(51) Int. Cl.: C07D 211/60

(54) **PROCESSES FOR THE MANUFACTURE OF HALOALKYL PYRIMIDYL COMPOUNDS, AND COMPOUNDS USEFUL IN THEIR MANUFACTURE**

(71) Applicant: SOLVAY SA, 1120 Brussels (BE)
(72) Inventor: The designation of the inventor has not yet been filed
(74) Representative: Mross, Stefan P.M.

(57) **Abstract**

The present invention concerns processes for the manufacture of haloalkyl pyrimidyl compounds, and compounds useful in their manufacture.

## Description

The present invention concerns processes for the manufacture of haloalkyl pyrimidyl compounds, and compounds useful in their manufacture.

Herbicides have attracted great attention over the past decades. Dimethyl 2-(difluoromethyl)-4-(2-methylpropyl)-6-(trifluoromethyl)pyridine-3,5-dicarbothioate, also known as Dithiopyr, is a representative of a class of haloalkyl substituted pyrimidyl compounds which has been found to inhibit root growth, by which emergence of weeds, in particular in turf and ornamental grasses, can be prevented or largely suppressed. Only few processes for the manufacture of Dithiopyr and related substances have been developed, for example as published in EP133612. Most processes rely on simultaneous introduction of two CF₃ groups, whereafter one CF₃ group has to be dehalogenated into a CF₂H group.

The present invention allows for new processes for the manufacture of haloalkyl substituted pyrimidyl compounds which allow for the asymmetric introduction of different haloalkyl groups onto a heterocyclic structure which can be converted into a haloalkyl substituted pridinyl compound. This eliminates, for example, the necessity for later removal of one or more halogen atoms from one of the haloalkyl groups. The processes according to the present invention start from generally well accessible starting materials, and often show good yields over several steps. The processes according to the present invention can have improved sustainability, for example, in selected cases, improved atom efficiency as halogen atoms, in particular fluorine atoms, need not to be removed later in the process. The compounds of the present invention are valuable starting materials and intermediates for chemical processes, for example in the processes according to the present invention.

The invention thus concerns a process for the manufacture of a compound of formula (I), which comprises a step of reacting a compound of formula (II), (III) and (IV) 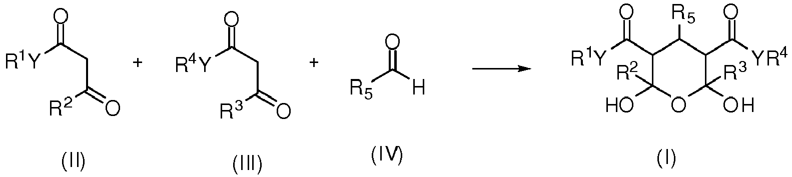 wherein R¹, R², R³, R⁴, R⁵ and Y are defined as below, and wherein R² or R³ are different.

The invention further concerns a process for the manufacture of a compound of formula (V), which comprises the process according to anyone of claim 1 to 7, and which comprises a step wherein compound (I) is contacted with an ammonia source, and wherein R¹, R², R³, R⁴, R⁵ and Y are defined as below, and wherein R² or R³ are different.

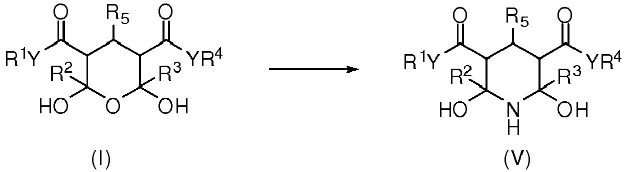

The invention further concerns a process for the manufacture of a compound of formula (VI), which comprises the process for the manufacture of a compound (I) and/or (V), and which comprises a step wherein compound (V) is contacted with a dehydration agent, and wherein R¹, R², R³, R⁴, R⁵ and Y are defined as below

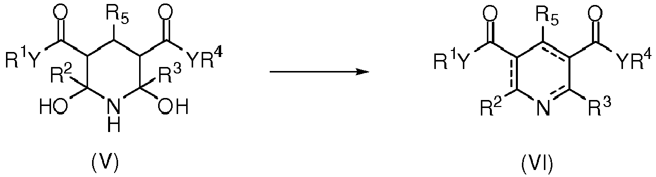

Another object of the present invention is a process for the manufacture of a compound of formula (VII), which comprises a process for the manufacture of a compound of formula (VI) according to the present invention, which comprises a step wherein a compound of formula (VI) is contacted with an oxidizing agent to obtain a compound of formula (VII), and wherein R¹, R², R³, R⁴, R⁵ and Y are defined as below

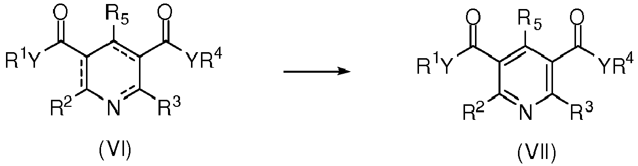

A further object of the present invention is a process for the manufacture of a compound of the formula of (I), (V), (VI) or (VII), which further comprises a step wherein a compound of formula (I), (V), (VI) or (VII), wherein Y is O and the other residues are defined below is converted into a compound wherein Y is S and the other residues are defined below, wherein the process comprises the step of saponifying a compound of (I), (V), (VI) or (VII), wherein Y is O and the other residues are defined below, to obtain a compound wherein Y is O and R¹ is H, contacting the obtained compound with a halogenating agent such that the group -OH is replaced with the group -X, wherein -X is F, Cl or Br, and contacting the obtained compound with at least one thiol HS-R⁶, wherein R⁶ is R¹ and R⁴, to obtain a compound of formula (I), (V), (VI) or (VII), wherein Y is S and the other residues are defined below.

Another object of the present invention concerns compounds of formula (I), wherein Y is S or O, wherein R¹, R², R³, R⁴, R⁵ and Y are defined as below, wherein R and R³ are different.

The invention concerns further a process for the manufacturing of an agriculturally or pharmaceutically active compound or a composition comprising an agriculturally or pharmaceutically active compound, which comprises at least one of the processes according to the present invention for the manufacture of a compound of formula (I), (V), (VI) and (VII) wherein the agriculturally active compound is preferably dimethyl 2-(difluoromethyl)-4-(2-methylpropyl)-6-(trifluoromethyl)pyridine-3,5-dicarbothioate or a precursor thereof.

In the present invention, designations in singular are in intended to include the plural; for example, "a solvent" is intended to denote also "more than one solvent" or "a plurality of solvents".
All aspects and embodiments of the present invention are combinable.

In the context of the present invention, the term "comprising" is intended to include the meaning of "consisting of'.

When a double bond is depicted in a particular E/Z geometry, this is intended to also denote the other geometric form as well as mixtures thereof.

In the context of the present invention, the term "C₁-C₄-alkyl group" is intended to denote straight or branched alkyl groups having one to four carbon atoms. This group comprises methyl, ethyl, n-propyl, isopropyl, n-, iso-, sec- and t-butyl. "C₁-C₈ alkyl group" intends to denote straight or branched alkyl groups having one to eight carbon atoms. This group comprises methyl, ethyl, n-propyl, isopropyl, n-, iso-, sec- and t-butyl, n-pentyl and its isomers, n-hexyl and its isomers, 1,3-dimethylbutyl, 3,3-dimethylbutyl, n-heptyl and its isomers and n-octyl and its isomers. Often, the C₁ to C₄ alkyl groups are the most preferred groups of the C₁-C₈ alkyl group. Similarly, the term "C₁-C₁₂-alkyl groups" is intended to denote straight or branched alkyl groups having one to twelve carbon atoms. Additionally to the groups comprised in the "C₁-C₈ alkyl group", the group comprises, for example, n-nonyl and its isomers, n-decyl and its isomers, n-undecyl and its isomers and n-dodecyl and its isomers. Often, the C₁ to C₄ alkyl groups are the most preferred groups of the C₁-C₁₂ alkyl group.

The term "C₃-C₈ cycloalkyl group" intends to denote mono-, bi- or tricyclic hydrocarbon groups comprising 3 to 10 carbon atoms, especially 3 to 6 carbon atoms. Examples of monocyclic groups include cyclopropyl, cyclobutyl, cyclopentyl, cyclohexyl, cycloheptyl or cyclooctyl. Examples of bicyclic groups include bicyclo[2.2.1]heptyl, bicyclo[3.1.1]heptyl, bicyclo[2.2.2]octyl and bicyclo[3.2.1]octyl. Examples of tricyclic groups are adamantyl and homoadamantyl.

The term "aryl group" intends to denote C₅-C₁₈ monocyclic and polycyclic aromatic hydrocarbons with 5 to 18 carbon atoms in the cyclic system. Specifically, this definition comprises, for example, the meanings cyclopentadienyl, phenyl, cycloheptatrienyl, cyclooctatetraenyl, naphthyl and anthracenyl.

Where indicated, a C₁-C₄ alkyl group, C₁-C₈ alkyl group, C₁-C₁₂ alkyl group and aryl group each can be optionally substituted by one or more substituents of the following group consisting of R', -X', -OR', -SR', -NR'₂,-SiR'₃, -COOR', -(C-O)R', -CN and -CONR'₂, wherein R' is selected independently, from the group consisting of hydrogen or C₁-C₁₂-alkyl group and X' is selected from the group consisting of F, Cl, Br, or I.

The present invention concerns in one aspect a process for the manufacture of a compound of formula (I), which comprises a step of reacting a compound of formula (II), (III) and (IV) 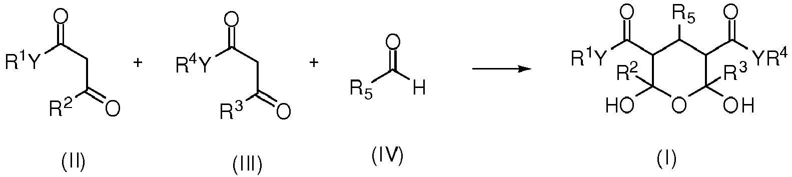
wherein R¹ is selected from the group consisting of H, an optionally substituted C₁-C₁₂ alkyl group, an optionally substituted C₃-C₈ cycloalkyl group, an optionally substituted aryl group,
wherein R² is selected from the group consisting of C₁-C₄-alkyl groups which is substituted by at least one halogen atom independently selected from the group consisting of F, Cl and Br or by a CF₃ group;
wherein R³ is selected from the group consisting of C₁-C₄-alkyl groups which is substituted by at least one halogen atom independently selected from the group consisting of F, Cl and Br or by a CF₃ group
wherein R⁴ is selected from the group consisting of H, an optionally substituted C₁-C₁₂ alkyl group, an optionally substituted C₃-C₈ cycloalkyl group, an optionally substituted aryl group
wherein R⁵ is selected from the group consisting of H, an optionally substituted C₁-C₁₂ alkyl group, an optionally substituted C₃-C₈ cycloalkyl group, an optionally substituted aryl group
wherein Y is S or O,
and wherein R² or R³ are different.

Generally, R² and R³ independently selected from the group consisting of C₁-C₄-alkyl groups which is substituted by at least one halogen atom independently selected from the group consisting of F, Cl and Br or by a CF₃ group. Preferably, R² and R³ are independently selected from the group consisting of CF₃, CF₂H, CCl₃, CCl₂H, CClF₂, CF₂Cl, CBr₃ and CBr₂H.

In a preferred aspect of the present invention, R³ is CF₂H and R² is CF₃.

In the process for the manufacture of a compound of formula (I), preferably R⁵ is a straight or branched C₁ to C₄ alkyl group, which is optionally substituted. R⁵ more preferably is the group -CH₂-CH(CH₃)₂. In another preferred aspect of the present invention, R⁵ is H.

In one aspect according to the process for the manufacture of (I), Y is S.

In another aspect according to the process for the manufacture of (I), Y is O.

In the process for the manufacture of a compound of formula (I), R¹ and R⁴ often are independently a straight or branched C₁ to C₄ alkyl group, which is optionally substituted, and wherein R¹ and R⁴ preferably are independently a methyl or an ethyl group. In one preferred aspect, R¹ and R⁴ are the same, and selected from methyl and ethyl, wherein ethyl is preferred.

The invention concerns father a process for the manufacture of a compound of formula (V), which comprises the process for the manufacture of a compound of formula (I) as described above, and which comprises a step wherein compound (I) is contacted with an ammonia source, and wherein R¹, R², R³, R⁴, R⁵ and Y are defined as above

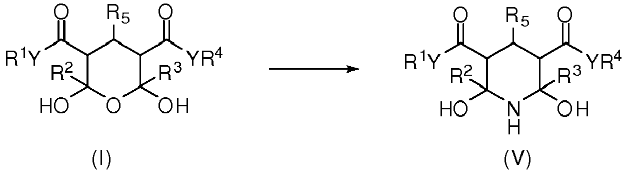

The ammonia source often is selected from NH₃ and a salt thereof, for example NH₄Cl, NH₄OAc or NH₄OH.

In one aspect according to the present invention, the process for the manufacture of a compound of formula (I) and the process for the manufacture of a compound of formula (V) is performed in a one-pot-procedure, wherein the ammonia source is already present when the compound of formula (II), (III) and (IV) are reacted. In this procedure, the compound of formula (V) is obtained directly when compounds of formula (II) and (III) are reacted.

The compound of formula (V) can be present in a mixture of its cis and trans isomer, as well as the cis and trans isomers only.

The invention further concerns a process for the manufacture of a compound of formula (VI), which comprises the process for the manufacture of a compound of formula (V), and which further comprises a step wherein compound (V) is contacted with a dehydration and wherein R¹, R², R³, R⁴, R⁵ and Y are defined as above:

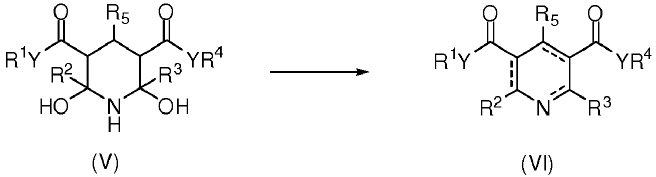

The dotted bonds in (VI) denote that more than isomers can be present in the compound of formula (VI), for example (VIa) and (VIb).

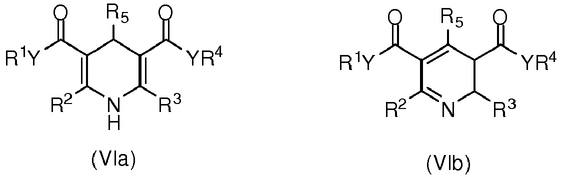

The term "dehydration agent" intends to denote an agent which is capable of eliminating water molecules from compound (V). Suitable dehydration agents are, for example, but not limited to, sulfuric acid, toluenesulfonic acid, orthoformates such as triethylorthoformate, zeolites, silica gel, NH₄OAc, molecular sieves and trifluoroacetic anhydride. The molecular sieves can be, in one aspect, packed into suitable packing materials, such as permeable membranes or fabrics, in order to allow for a steady flow of the reaction mixture while keeping the molecular sieves intact. In another aspect, at least one solid dehydrating agent, such as zeolites, silica gel, or molecular sieves, can be packed into a permeable container, such as a packed column, and the reaction mixture is looped through the container comprising the dehydration agent. The term "compound of formula (VI)" denotes any mixture of any isomer of (VI), but also denotes (VI) in only one isomeric form. Suitable reaction media for the reaction to obtain (VI) from (V) are, for example, halogenated hydrocarbons, such as methylene chloride, hydrocarbons, or optionally substituted aromatic hydrocarbons, such as toluenes.

Another object of the present invention is a process for the manufacture of a compound of formula (VII), which comprises the process for the manufacture of compound (VI) as described above, which comprises a step wherein a compound of formula (VI) is contacted with an oxidizing agent to obtain a compound of formula (VII), and wherein R¹, R², R³, R⁴, R⁵ and Y are defined as above

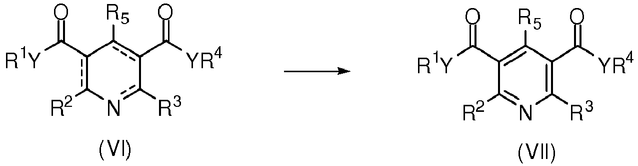

Again, the dotted lines in compound (VI) denote that two double bonds are present in the heterocyclic ring of (VI), which allow for two isomeric forms of (VI) to be present. Appropriate oxidizing agents include, for example, 1, 8-diazabicyclo-[5. 4. 0]- undec-5-ene (DBU), tributylamine, O₂, optionally in the presence of a catalyst, quinone derivatives such as chloranil, benzoquinones or anthraquinone or sodium nitrite. The step of contacting the compound of formula (VI) with an oxidizing agent can be performed in at least one solvent or without a solvent. Suitable solvents include, for example, cyclic and acyclic ethers, for example, tetrahydrofurane, diethylether and dioxane, carboxylic acids, such as acetic acid, halogenated hydrocarbons, such as methylene chloride, hydrocarbons, or optionally substituted aromatic hydrocarbons, such as toluenes.

The invention concerns also a process for the manufacture of a compound of formula (I), (V), (VI) or (VII) as described above, which further comprises a step wherein a compound of formula (I), (V), (VI) or (VII), wherein R² and R³ are defined as before 10, wherein Y is O and R⁴ and R¹ is selected from the group consisting an optionally substituted C₁-C₁₂ alkyl group, an optionally substituted C₃-C₈ cycloalkyl group and an optionally substituted aryl group, wherein R¹ and R⁴ preferably are an optionally substituted C₁ to C₄ alkyl group, is converted into a compound wherein Y is S and R⁴ and R¹ is selected from the group consisting an optionally substituted C₁-C₁₂ alkyl group, an optionally substituted C₃-C₈ cycloalkyl group and an optionally substituted aryl group, wherein R¹ and R⁴ preferably are an optionally substituted C₁ to C₄ alkyl group, wherein the process comprises the step of saponifying a compound of formula (I), (V), (VI) or (VII), wherein Y is O and R¹ is selected from the group consisting an optionally substituted C₁-C₁₂ alkyl group, an optionally substituted C₃-C₈ cycloalkyl group and an optionally substituted aryl group, wherein R¹ and R⁴ preferably are an optionally substituted C₁ to C₄ alkyl group, to obtain a compound wherein Y is O and R¹ is H, 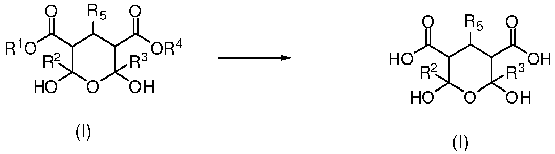 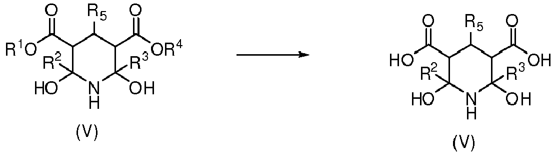 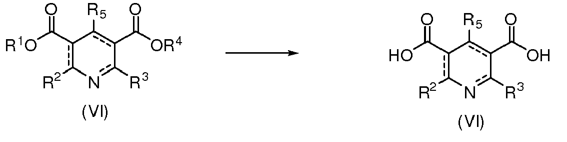 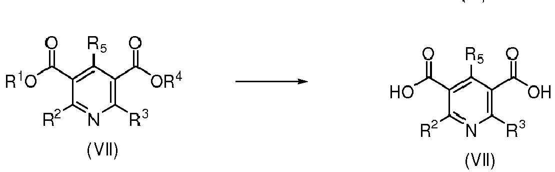 contacting the obtained compound with a halogenating agent such that the group -OH is replaced with the group -X, wherein -X is F, Cl or Br, wherein X preferably is Cl 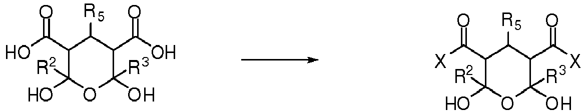 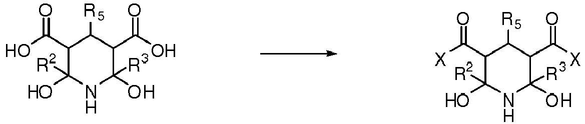 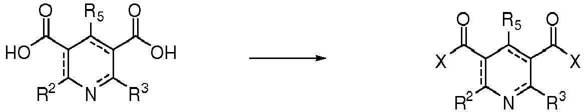 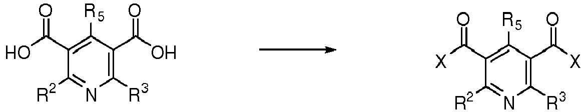 and contacting the obtained compound with at least one thiol HS-R⁶, wherein R⁶ is R¹ and R⁴, to obtain a compound of formula (I), (V), (VI) or (VII), wherein Y is S and R⁴ and R¹ is selected from the group consisting an optionally substituted C₁-C₁₂ alkyl group, an optionally substituted C₃-C₈ cycloalkyl group and an optionally substituted aryl group, wherein R¹ and R⁴ preferably are an optionally substituted C₁ to C₄ alkyl group.

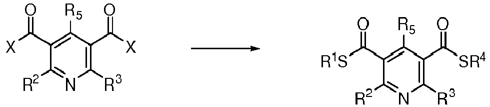

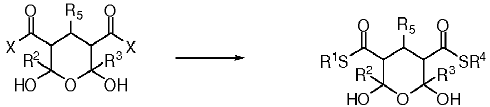

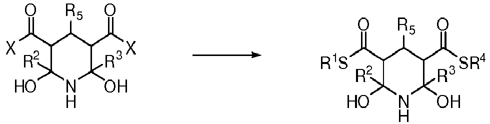

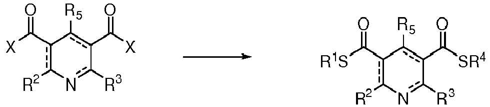

The halogenating agent is a halogenating agent which is capable of transforming a carboxylic acid group into a carboxylic acid halide group, such as SOCl₂ and oxalyl chloride. The thiol HS-R⁶ is preferably selected from methane thiole or ethane thiole, which is preferably added to the reaction in a salt form, such as methanethiol sodium salt or ethanethiol sodium salt.

The present invention further concerns compounds of formula (I), wherein Y is S or O, wherein R¹ is selected from the group consisting of H, an optionally substituted C₁-C₁₂ alkyl group, an optionally substituted C₃-C₈ cycloalkyl group, an optionally substituted aryl group, wherein R² is selected from the group consisting of C₁-C₄-alkyl groups which is substituted by at least one halogen atom independently selected from the group consisting of F, Cl and Br or by a CF₃ group, wherein R³ is selected from the group consisting of C₁-C₄-alkyl groups which is substituted by at least one halogen atom independently selected from the group consisting of F, Cl and Br or by a CF₃ group, R⁴ is selected from the group consisting of H, an optionally substituted C₁-C₁₂ alkyl group, an optionally substituted C₃-C₈ cycloalkyl group, an optionally substituted aryl group, and R⁵ is selected from the group consisting of H, an optionally substituted C₁-C₁₂ alkyl group, an optionally substituted C₃-C₈ cycloalkyl group, an optionally substituted aryl group, and wherein R² and R³ are different. Most preferably in compounds of formula (I), R² is selected from the group consisting of CF₃, CBr₃ and CCl₃ and R³ is selected from the group consisting of CF₂H, CBr₂H and CCl₂H.

The following non-restrictive list indicates the most preferable compounds of formula (I):

| formula | R¹ | R² | R³ | R⁴ | R⁵ | Y |
|---|---|---|---|---|---|---|
| I | Me | CF₂H | CF₃ | Me | CH(CH₃)₂ | S |
| I | Me | CF₃ | CF₂H | Me | CH(CH₃)₂ | S |
| I | Et | CF₂H | CF₃ | Et | CH(CH₃)₂ | S |
| I | Et | CF₃ | CF₂H | Et | CH(CH₃)₂ | S |
| I | Me | CF₂H | CF₃ | Me | CH(CH₃)₂ | O |
| I | Me | CF₃ | CF₂H | Me | CH(CH₃)₂ | O |
| I | Et | CF₂H | CF₃ | Et | CH(CH₃)₂ | O |
| I | Et | CF₃ | CF₂H | Et | CH(CH₃)₂ | O |

These compounds of formula (I) as described above are useful as starting materials for compounds of formula (V), (VI) and (VII) which are pharmaceutically or agrochemically active ingredients such as dimethyl 2-(difluoromethyl)-4-(2-methylpropyl)-6-(trifluoromethyl)pyridine-3,5-dicarbothioate, or for precursors of such pharmaceutically or agrochemically active ingredients.

When R⁵ is H, the invention further concerns a process for the manufacture of a compound of formula (VII) wherein R⁵ is -CH(CH₃)₂, which comprise any of the processes according to the present invention, and which further comprises a step wherein a compound of formula (VII) wherein R⁵ is H is converted into a compound of formula (VII) wherein R⁵ is -CH(CH₃)₂ by reaction with isobutylene in the presence of a Friedel Crafts catalyst, such as, for example, CuI, or a solid phase reaction catalysed, for example, by solid phosphoric acid as described in M. Cowley, Ind. Eng. Chem. Res. 2006, 45, 7399-7408.

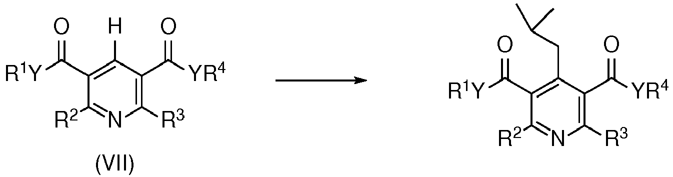

The invention concerns further a process for the manufacturing of an agriculturally or pharmaceutically active compound or a composition comprising an agriculturally or pharmaceutically active compound, which comprises at least one of the processes according to the present invention for the manufacture of a compound of formula (I), (V), (VI) or (VII) wherein the agriculturally active compound is preferably dimethyl 2-(difluoromethyl)-4-(2-methylpropyl)-6-(trifluoromethyl)pyridine-3,5-dicarbothioate or a precursor thereof.

The invention also concerns a compound of formula (III), wherein R³ is selected from the group consisting of CF₂H, CBr₂H and CCl₂H, Y is S and R⁴ is selected from the group consisting an optionally substituted C₁-C₁₂ alkyl group, an optionally substituted C₃-C₈ cycloalkyl group and an optionally substituted aryl group, wherein R⁴ preferably is Me or Et.

Another object of the present invention is the use of a compound according to the present invention in a process for the manufacturing of an agriculturally or pharmaceutically active compound or a composition comprising an agriculturally or pharmaceutically active compound, preferably dimethyl 2-(difluoromethyl)-4-(2-methylpropyl)-6-(trifluoromethyl)pyridine-3,5-dicarbothioate or a precursor thereof.

Should the disclosure of any patents, patent applications, and publications which are incorporated herein by reference conflict with the description of the present application to the extent that it may render a term unclear, the present description shall take precedence.

The following examples are intended to further explain the invention without limiting it.

The starting materials for the processes and steps according to the present invention can be obtained from commercial sources or can be manufactured according to procedures known from the public domain. Butanoic acid, 4,4,4-trifluoro-3-oxo-, ethyl ester (Ethyl 4,4,4-trifluoroacetoacetate) is for example known from EP206953. Butanoic acid, 4,4-difluoro-3-oxo-, ethyl ester (Ethyl 4, 4-difluoroacetoacetate) is for example known from IT1401696. The respective carbothioates can be obtained, for example, by saponification, halogenation and reaction with thiols starting from Ethyl 4,4-difluoroacetoacetate or Ethyl 4,4,4-trifluoroacetoacetate. The preparation of methyl-4,4,4-trifluoro-3-oxo-butanethioate is known from US4785129. Methyl-4,4-difluoro-3-oxo-butanethioate can be prepared according to a similar procedure starting from difluoroacetyl chloride.

### Example 1 diethyl 2-(difluoromethyl)-2,6-dihydroxy-4-isobutyl-6-(trifluoromethyl)tetrahydro-2H-pyran-3,5-dicarboxylate

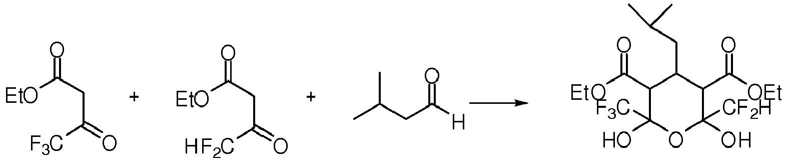

A 500 ml single necked flask is charged with 0. 278 mole of isovaleraldehyde, 0.278 mole of ethyl 4,4,4-trifluoro-3-oxobutanoate , 0.278 mole of ethyl 4,4-difluoro-3-oxobutanoate and approximately 150 ml of ethanol. To this is added 3 g (0. 0156 mole) of potassium fluoride. The mixture is stirred at room temperature for 18 hours. The material is concentrated and diluted with ethyl ether. The organics are washed with water, dried and concentrated to give crude diethyl 2-(difluoromethyl)-2,6-dihydroxy-4-isobutyl-6-(trifluorome-thyl)-tetrahydro-2H-pyran-3,5-dicarboxylate.

S,S-dimethyl 2-(difluoromethyl)-2,6-dihydroxy-4-isobutyl-6-(trifluoromethyl)tetrahydro-2H-pyran-3,5-bis(carbothioate) is obtained according to a comparable procedure starting from S-methyl 4,4,4-trifluoro-3-oxobutanethioate, S-methyl 4,4-difluoro-3-oxobutanethioate and butyraldehyde.

### Example 2 diethyl 2-(difluoromethyl)-2,6-dihydroxy-4-isobutyl-6-(trifluoromethyl)piperidine-3,5-dicarboxylate

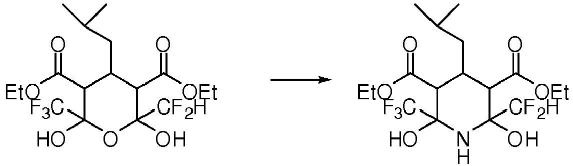

A 500 ml round bottomed flask was charged with 150 - 200 mL of ethanol and 0. 0909 mole diethyl 2-(difluoromethyl)-2,6-dihydroxy-4-isobutyl-6-(trifluoromethyl)-tetrahydro-2H-pyran-3,5-dicarboxylate. The mixture is stirred by a magnetic stirrer while 8. 23 g (0. 136 mole) of 58% aqueous ammonium hydroxide is added slowly to the flask. The mixture is stirred for 18 hours under nitrogen. The precipitate is filtered, washed and dried to obtain crude diethyl 2-(difluoromethyl)-2,6-dihydroxy-4-isobutyl-6-(trifluoromethyl)-piperidine-3,5-dicarboxylate.

### Example 3 diethyl 2-(difluoromethyl)-4-isobutyl-6-(trifluoromethyl)-dihydropyridine-3,5-dicarboxylate

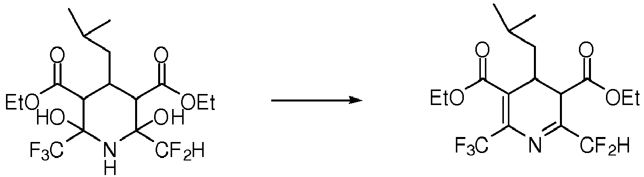

To an ice water cooled mixture of 20 ml of concentrated sulfuric acid and 20 ml of methylene chloride is added 0.058 mole Diethyl 2-(difluoromethyl)-2,6-dihydroxy-4-isobutyl-6-(trifluoromethyl)-piperidine-3,5-dicarboxylate. The reaction mixture is stirred for 20 minutes and poured into 300 mL of ice water. The methylene chloride layer is separated and washed once with 30 ml of saturated sodium bicarbonate, dried and concentrated to give the crude product as an isomeric mixture, one of which is shown above.

Diethyl 2-(difluoromethyl)-4-isobutyl-6-(trifluoromethyl)-dihydropyridine-3,5-bis(carbothioate) is obtained according to a comparable procedure.

### Example 4 Diethyl 2-(difluoromethyl)-4-isobutyl-6-(trifluoromethyl)pyridine-3,5-dicarboxylate

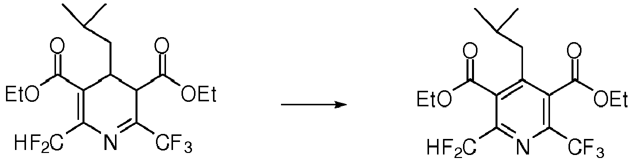

An isomeric mixture of 0.4 moles of diethyl 2-(difluoromethyl)-4-isobutyl-6-(trifluoromethyl)pyridine-3,5-dicarboxylate, 0.4 moles of 1, 8-diazabicyclo-[5. 4. 0]- undec-5-ene (DBU), and 50 ml of tetrahydrofuran is held at reflux for 19 hours, cooled and poured into a mixture of 300 g of ice and 25 ml of concentrated hydrochloric acid. The organic layer is separated and the aqueous layer extracted with 50 ml of CH₂Cl₂ twice. The combined organic materials are dried (MgSO4) and concentrated.

Diethyl 2-(difluoromethyl)-4-isobutyl-6-(trifluoromethyl)pyridine-3,5-dicarboxylate is obtained according to a comparable procedure.

### Example 5 2-(difluoromethyl)-4-isobutyl-6-(trifluoromethyl)pyridine-3,5-dicarboxylic acid

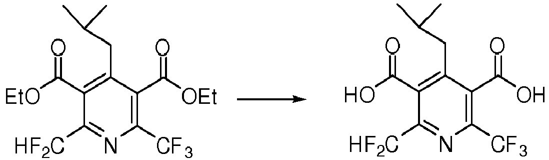

A 500-mL flask is charged with 0.082 mole of diethyl 2-(difluoromethyl)-4-isobutyl-6-(trifluoromethyl)pyridine-3,5-dicarboxylate and 100 ml of methyl alcohol. In another flask, 75 ml of water and 10,76 g (0.163 mole) of potassium hydroxide are combined. The aqueous KOH is poured into the 500-mL flask and the mixture is heated to reflux overnight. The reaction mixture is cooled and extracted once with ethyl ether. The aqueous layer is acidified with concentrated hydrochloric acid and extracted with ethyl ether. The organics are dried on anhydrous magnesium sulphate, filtered, and concentrated to yield the crude 2-(difluoromethyl)-4-isobutyl-6-(trifluoromethyl)pyridine-3,5-dicarboxylic acid.

### Example 6 2-(difluoromethyl)-4-isobutyl-6-(trifluoromethyl)pyridine-3,5-dicarbonyl dichloride

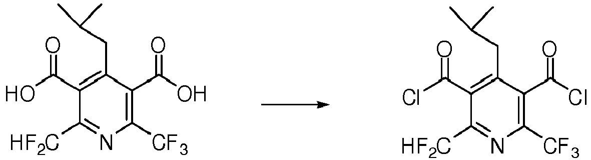

A mixture of 0.105 mole of the product of 2-(difluoromethyl)-4-isobutyl-6-(trifluoromethyl)pyridine-3,5-dicarboxylic acid and 50 ml of thionyl chloride is held at reflux for 18 hours and concentrated in vacuo to give the crude 2-(difluoromethyl)-4-isobutyl-6-(trifluoromethyl)pyridine-3,5-dicarbonyl dichloride.

### Example 7 S,S-dimethyl 2-(difluoromethyl)-4-isobutyl-6-(trifluoromethyl)-pyridine-3,5-bis(carbothioate)

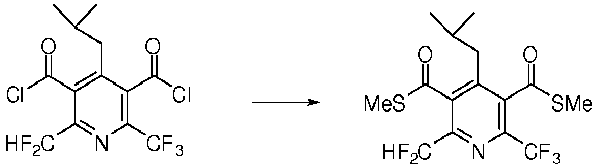

A mixture of 1,23 mmole of 2-(difluoromethyl)-4-isobutyl-6-(trifluoromethyl)pyridine-3,5-dicarbonyl dichloride and 2 ml of dichloromethane was added drop wise to a mixture of 106 mg of sodium methyl mercaptan and 1 ml of dichloromethane at 0 degrees centigrade The reaction mixture was stirred for 3.5 hours at 0 degrees centigrade. The mixture was concentrated under reduced pressure, ethyl acetate was added to the obtained residues, and insoluble matter was separated by filtration. The filtrate was concentrated to yield the crude S,S-dimethyl 2-(difluoromethyl)-4-isobutyl-6-(trifluoromethyl)-pyridine-3,5-bis(carbothioate).

## Claims

1. Process for the manufacture of a compound of formula (I), which comprises a step of reacting a compound of formula (II), (III) and (IV) 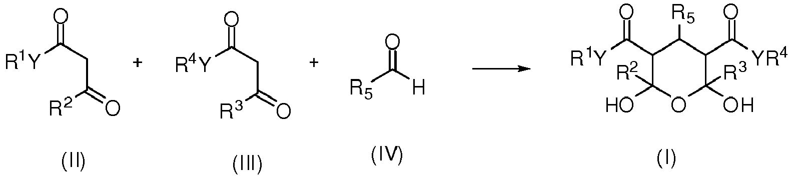
wherein R¹ is selected from the group consisting of H, an optionally substituted C₁-C₁₂ alkyl group, an optionally substituted C₃-C₈ cycloalkyl group, an optionally substituted aryl group
wherein R² is selected from the group consisting of C₁-C₄-alkyl groups which is substituted by at least one halogen atom independently selected from the group consisting of F, Cl and Br or by a CF₃ group
wherein R³ is selected from the group consisting of C₁-C₄-alkyl groups which is substituted by at least one halogen atom independently selected from the group consisting of F, Cl and Br or by a CF₃ group
wherein R⁴ is selected from the group consisting of H, an optionally substituted C₁-C₁₂ alkyl group, an optionally substituted C₃-C₈ cycloalkyl group, an optionally substituted aryl group
wherein R⁵ is selected from the group consisting of H, an optionally substituted C₁-C₁₂ alkyl group, an optionally substituted C₃-C₈ cycloalkyl group, an optionally substituted aryl group
wherein Y is S or O,
and wherein R² or R³ are different.

2. Process according to claim 1, wherein R² and R³ are selected from the group consisting of CF₃, CF₂H, CCl₃, CCl₂H, CClF₂, CF₂Cl, CBr₃ and CBr₂H.

3. Process according to claim 2, wherein R² is CF₃ and R³ is CF₂H.

4. Process according to anyone of claims 1 to 3, wherein Y is S.

5. Process according to anyone of claims 1 to 3, wherein Y is O.

6. Process according to anyone of claims 1 to 5, wherein R⁵ is a straight or branched C₁ to C₄ alkyl group, which is optionally substituted, wherein R⁵ preferably is the group -CH₂-CH(CH₃)₂.

7. Process according to anyone of claims 1 to 6, wherein R¹ and R⁴ independently are a straight or branched C₁ to C₄ alkyl group, which is optionally substituted, and wherein R¹ and R⁴ preferably are independently a methyl or an ethyl group.

8. Process for the manufacture of a compound of formula (V), which comprises the process according to anyone of claim 1 to 7, and which comprises a step wherein compound (I) is contacted with an ammonia source, and wherein R¹, R², R³, R⁴, R⁵ and Y are defined as in anyone of claim 1 to 7

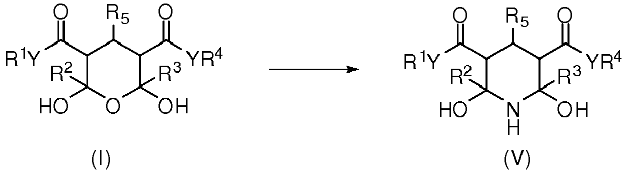

9. Process for the manufacture of a compound of formula (VI), which comprises the process according to anyone of claim 1 to 8, and which comprises a step wherein compound (V) is contacted with a dehydration agent, and wherein R¹, R², R³, R⁴, R⁵ and Y are defined as in anyone of claim 1 to 8

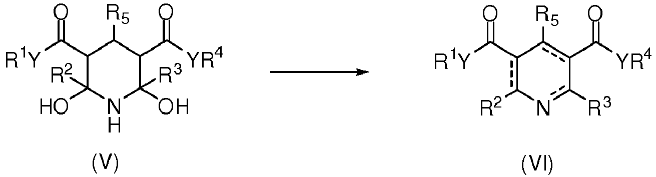

10. Process for the manufacture of a compound of formula (VII), which comprises the process according to claim 9, which comprises a step wherein a compound of formula (VI) is contacted with an oxidizing agent to obtain a compound of formula (VII), and wherein R¹, R², R³, R⁴, R⁵ and Y are defined as in anyone of claim 1 to 9

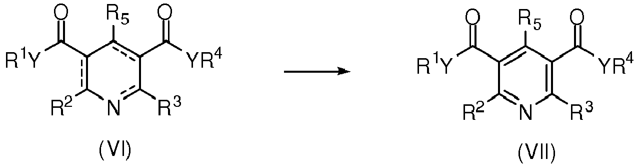

11. Process according to anyone of claims 1 to 10, which further comprises a step wherein a compound of formula (I), (V), (VI) or (VII), wherein R² and R³ are defined as in anyone of claims 1 to 10, wherein Y is O and R⁴ and R¹ is selected from the group consisting an optionally substituted C₁-C₁₂ alkyl group, an optionally substituted C₃-C₈ cycloalkyl group and an optionally substituted aryl group, wherein R¹ and R⁴ preferably are an optionally substituted C₁ to C₄ alkyl group, is converted into a compound wherein Y is S and R⁴ and R¹ is selected from the group consisting an optionally substituted C₁-C₁₂ alkyl group, an optionally substituted C₃-C₈ cycloalkyl group and an optionally substituted aryl group, wherein R¹ and R⁴ preferably are an optionally substituted C₁ to C₄ alkyl group, wherein the process comprises the step of saponifying a compound of formula (I), (V), (VI) or (VII), wherein Y is O and R¹ is selected from the group consisting an optionally substituted C₁-C₁₂ alkyl group, an optionally substituted C₃-C₈ cycloalkyl group and an optionally substituted aryl group, wherein R¹ and R⁴ preferably are an optionally substituted C₁ to C₄ alkyl group, to obtain a compound wherein Y is O and R¹ is H, contacting the obtained compound with a halogenating agent such that the group -OH is replaced with the group -X, wherein -X is F, Cl or Br, and contacting the obtained compound with at least one thiol HS-R⁶, wherein R⁶ is R¹ and R⁴, to obtain a compound of formula (I), (V), (VI) or (VII), wherein Y is S and R⁴ and R¹ is selected from the group consisting an optionally substituted C₁-C₁₂ alkyl group, an optionally substituted C₃-C₈ cycloalkyl group and an optionally substituted aryl group, wherein R¹ and R⁴ preferably are an optionally substituted C₁ to C₄ alkyl group.

12. Compound of formula (I), wherein Y is S or O,
wherein R¹ is selected from the group consisting of H, an optionally substituted C₁-C₁₂ alkyl group, an optionally substituted C₃-C₈ cycloalkyl group, an optionally substituted aryl group, wherein R² is selected from the group consisting of C₁-C₄-alkyl groups which is substituted by at least one halogen atom independently selected from the group consisting of F, Cl and Br or by a CF₃ group, wherein R³ is selected from the group consisting of C₁-C₄-alkyl groups which is substituted by at least one halogen atom independently selected from the group consisting of F, Cl and Br or by a CF₃ group, R⁴ is selected from the group consisting of H, an optionally substituted C₁-C₁₂ alkyl group, an optionally substituted C₃-C₈ cycloalkyl group, an optionally substituted aryl group, and R⁵ is selected from the group consisting of H, an optionally substituted C₁-C₁₂ alkyl group, an optionally substituted C₃-C₈ cycloalkyl group, an optionally substituted aryl group, and wherein R² and R³ are different.

13. Process for the manufacturing of an agriculturally or pharmaceutically active compound or a composition comprising an agriculturally or pharmaceutically active compound, which comprises at least one of the process according to anyone of claims 1 to 11.

14. Compound of formula (III), wherein R³ is selected from the group consisting of CF₂H, CBr₂H and CCl₂H, Y is S and R⁴ is selected from the group consisting an optionally substituted C₁-C₁₂ alkyl group, an optionally substituted C₃-C₈ cycloalkyl group and an optionally substituted aryl group, wherein R⁴ preferably is Me or Et.

15. Use of a compound according to claim 12 or 13 in a process for the manufacturing of an agriculturally or pharmaceutically active compound or a composition comprising an agriculturally or pharmaceutically active compound, preferably dimethyl 2-(difluoromethyl)-4-(2-methylpropyl)-6-(trifluoromethyl)pyridine-3,5-dicarbothioate or a precursor thereof.
